# EUROPEAN PATENT APPLICATION

(11) **EP 4 417 211 A1**
(43) Date of publication of application: **21.08.2024**
(21) Application number: 24158158.6
(22) Date of filing: 16.02.2024
(51) Int. Cl.: A61K 35/17, A61P 35/00, C12N 5/0783

(54) **PHARMACEUTICAL COMPOSITION COMPRISING MODIFIED NATURAL KILLER CELLS AND ANTIGEN-SPECIFIC T CELLS, MANUFACTURING METHOD THEREOF, AND METHOD OF USING THE SAME**

(30) Priority: 17.02.2023 US 202318170671
(71) Applicant: Fullhope Biomedical Co., Ltd., New Taipei City 24159 (TW); National Yang Ming Chiao Tung University, Taipei City 112304 (TW)
(72) Inventor: LEE, Jan-Mou, 24159 New Taipei City (TW); LAN, Keng-Li, 112304 Taipei City (TW); FANG, Chih-Hao, 24159 New Taipei City (TW); CHENG, Ya-Fang, 24159 New Taipei City (TW)
(74) Representative: Engelhard, Markus

(57) **Abstract**

This disclosure provides method of manufacturing pharmaceutical composition for treating cancer, which provides great cell yield via not performing negative selection on CD3⁻CD19⁻CD14⁻. By administration of the pharmaceutical composition, cancer cells in a subject may be effectively inhibited via cell-mediated immunity.

## Description

### FIELD OF INVENTION

The disclosure relates to pharmaceutical compositions comprising modified natural killer (NK) cells and antigen-specific T cells. Methods are also provided for culturing of the modified NK cells and antigen-specific T cells.

### BACKGROUND OF THE INVENTION:

Immune surveillance plays a critical role against cancer and represents a very attractive therapeutic approach, especially in light of the many shortcomings of conventional surgery, radiation and chemotherapies in the management of cancer.

The human body's first line of defense against cancer is the natural killer (NK) cell, with the phenotype of CD3⁻CD14⁻CD19⁻CD56⁺CD16⁺NGK2D⁺CD11c^{dim}HLA-DR⁻CD86⁻ CD83⁻. NK cells are cytotoxic lymphocytes that actively scan the body for abnormal cells, destroying them before they can develop into actual cancer cells. As NK cells patrol the body, they interact with many types of cells using their array of activating and inhibiting surface receptors. Most cancer cells engage the NK cell's activating receptors, which triggers its natural kill response.

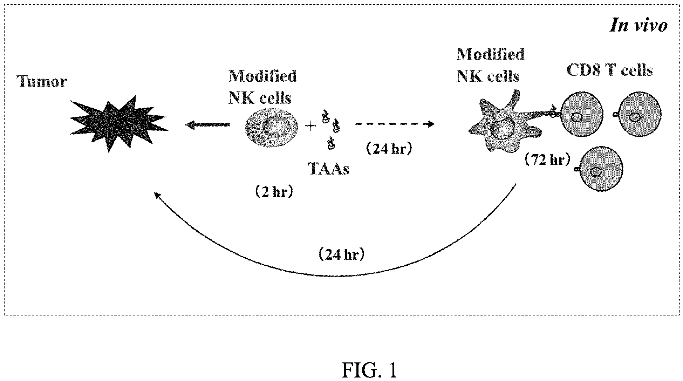

US20210260115A1 disclosed modified NK cells possessing both NK cell function and dendritic cell function and method of culturing the same. When administering the modified NK cells to the subject with cancer, the modified NK cells kills tumor cells and presents tumor-associated antigen (TAA) to naive T cells in the subject, and delivers T cell responses *in vivo* (FIG. 1). However, a step of negative selection of CD3⁻CD14⁻CD19⁻ mononuclear cells prior to the culturing step is needed, which leads to the problem of high cost and complicated manufacturing process due to low cell yield. Nonetheless, there is also uncertainty towards the efficacy of TAA-specific CD8 T cells induced by the modified NK cells *in vivo.*

These findings support a rationale for developing a NK based therapy against cancer cells and culture methods to generate greater number of therapeutically competent NK cells for clinical applications, as there is still an unmet need for effective treatment and/or prevention for cancer. The present disclosure provides pharmaceutical compositions comprising modified natural killer cells and antigen-specific T cells, and manufacturing method thereof.

### SUMMARY OF THE INVENTION

In view of the urgent need of the art, provided herein are method of manufacturing a pharmaceutical compositions comprising modified natural killer (NK) cells and antigen-specific T cells that are safe and effective in the treatment of the cancer. In addition, the modified NK cells and the antigen-specific T cells are easy to manufacture with low cost and high yield.

In one embodiment, the present disclosure provides a method of manufacturing pharmaceutical composition for treating cancer, comprising
obtaining mononuclear cells from a subject with cancer;
contacting the mononuclear cells with a first culturing medium comprising IL-15, IL-12 and IL-18 for about 1-6 day(s) to obtain a cultured cell population; and
contacting the cultured cell population with a second culturing medium comprising IL-15 and IL-12 for about 1-6 day(s) after contacting with the first culturing medium;
wherein a negative selection of CD3⁻CD19⁻CD14⁻ is not performed on the mononuclear cells prior to the contact step.

In one embodiment, the present disclosure provides a pharmaceutical composition comprising the modified NK cell with a phenotype of CD45⁺CD3⁻CD19⁻CD14⁻; antigen-specific T cells with a phenotype of 4-1BB⁺CD8⁺; and pharmaceutically acceptable carrier or excipient. In a preferred embodiment, the modified NK cell may further comprise a phenotype of CDS6^{hi}CD16^{dim}NKG2D⁺CD11c⁺CD86⁺HLA-DR⁺CD83⁻HLA-ABC⁺, *i.e.* the modified NK cell may comprise a phenotype of CD45⁺CD3⁻CD19⁻CD14⁻ CDS6^{hi}CD16^{dim}NKG2D⁺CD11c⁺CD86⁺HLA-DR⁺CD83⁻HLA-ABC⁺. In an embodiment, the antigen-specific T cells cell may further comprise a phenotype of CD25^{hi}CD27⁻CD28⁻. In another embodiment, the antigen-specific T cells cell may comprise a phenotype of Perforin^{dim}Ganzyme B^{dim}CD107a⁺TNF-α⁺IFN-γ^{dim}. In a preferred embodiment, the modified NK cell may further comprise a phenotype of CD25^{hi}CD27⁻CD28⁻4-1BB⁺Perforin^{dim}Ganzyme B^{dim}CD107a⁺TNF-α⁺IFN-γ^{dim}.

Some embodiments provide pharmaceutical compositions which are manufactured by the method described herein.

Some embodiments provide a method of treating cancer cells, comprising administering an effective amount of pharmaceutical compositions as described herein to a subject in need thereof. Likewise some embodiments provide a pharmaceutical composition as described herein, for use in a method of treating cancer, said method comprising administering an effective amount of a pharmaceutical composition as described herein to a subject in need thereof.

In a preferred embodiment, the effective amount may be about l×10³ to about l×10⁹ cells per dose.

In a preferred embodiment, the modified NK cell or the antigen-specific T cell may be autologous or allogeneic.

In a preferred embodiment, the modified NK cell or the antigen-specific T cell may be derived from peripheral blood, cord blood or bone marrow.

In a preferred embodiment, the method may further comprise expanding the modified NK cell with the antigen-specific T cells *in vitro.*

In a preferred embodiment, the first culturing medium and the second culturing medium may further comprise a hematopoietic cell medium, preferably an AIM-V medium.

In a preferred embodiment, the first culturing medium and the second culturing medium may further comprise a serum protein, preferably a human platelet lysate.

The culturing methods described herein allow for isolation of a greater number of modified NK cells from a fixed amount of a sample, for example, 10 mL of blood.

### BRIEF DESCRIPTION OF THE DRAWINGS

Illustrative embodiments of the present application are described in detail below with reference to the following figures:
FIG. 1 illustrates that the tumor-associated antigens (TAAs) were presented to CD8 T cell by the modified NK cells *in vivo* as in the prior art, US20210260115A1. The adoptive cell transfer of modified NK cells mediate NK-dependent cytotoxicity to kill tumor cells and result in release of tumor-associated antigens (TAAs). Meanwhile, the modified NK cells also acquire tumor antigens and present the full spectrum of TAAs to CD8 T cells, consequently inducing multiple-epitope tumor-specific CD8 T cell proliferation and activation. These tumor-specific T cells can further migrate to tumor-bedding sites to mount long-lasting anti-tumor T cell responses *in vivo.*

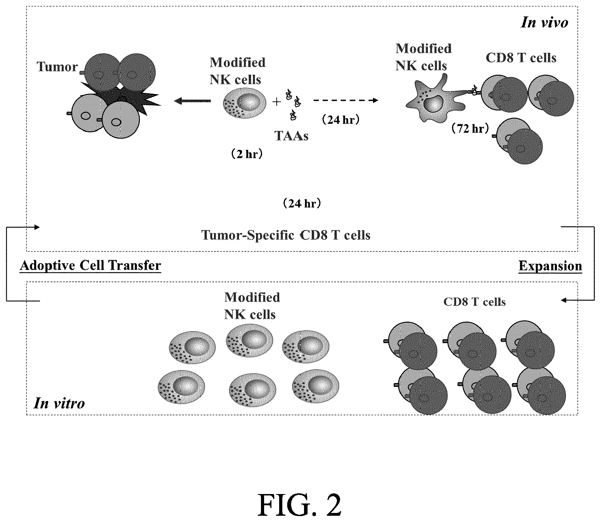

FIG. 2 illustrates that the tumor-associated antigens (TAAs) were presented to CD8 T cell by the modified NK cells *in vivo* and the antigen-specific CD8 T cell was expanded *in vitro* and adoptively transfer back to the patient to be treated. The pharmaceutical composition comprises modified NK cells and tumor-specific CD8 T cells, i.e. antigen-specific T cells. Modified NK cells can activate tumor-specific CD8 T cells *in-vivo* via antigen-presenting cell activity, which can be further expanded in the manufacture of modified NK cells. Comparing to those described in FIG.1, the adoptive cell transfer of the present disclosure retains the original characteristic of modified NK cells to drive multiple-epitope tumor-specific CD8 T cell activation. In addition, it also delivers robust anti-tumor Cytotoxic T lymphocyte (CTL) cell responses through modified NK cell-driven CD8 T cells that expanded *in vitro.* This novel pharmaceutical composition's repeated adoptive cell transfer can deliver up-to-date anti-tumor immune responses.
FIG. 3 illustrates the absolute cell numbers of the modified NK cells cultured in accordance with the method of prior art and that of the present disclosure.
FIG. 4 illustrates that the method of the present disclosure significantly expanded CD8 T cell population comparing to fresh peripheral blood mononuclear cells (PBMCs).
FIGs. 5A and 5B illustrate that the method of the present disclosure significantly expanded CD8 T cell population and increased CD8 to CD4 ratio.
FIG. 6 illustrates that the method of the present disclosure induced antigen (Ag)-specific activation-induced markers (AIM)⁺ CD8 T cells.
FIG. 7 illustrates that the method of the present disclosure induced CD8 T cell expressing Ag-specific T cell phenotype.
FIG. 8 illustrates that the method of the present disclosure induced degranulation of CD8 T cells.
FIG. 9 illustrates that the method of the present disclosure induced CD8 T cell to deliver degranulation molecules, including Perforin, Granzyme B, CD107a, TNF-α and IFN-γ.
FIGs. 10 and 11 illustrate that the pharmaceutical composition manufactured in accordance with the method of the present disclosure exhibited tumoricidal activity against primary epithelial ovarian cancer (EOC) cells.
FIG. 12 is a flow chart in accordance with an embodiment of the methods of manufacturing the pharmaceutical compositions.

### DETAILED DESCRIPTION

The foregoing and other aspects of the present disclosure will now be described in more detail with respect to other embodiments described herein. It should be appreciated that the invention can be embodied in different forms and should not be construed as limited to the embodiments set forth herein. Rather, these embodiments are provided so that this disclosure will be thorough and complete, and will fully convey the scope of the invention to those skilled in the art.

The terminology used in the description of the invention herein is for the purpose of describing particular embodiments only and is not intended to be limiting of the invention. As used in the description of the invention and the appended claims, the singular forms "a", "an" and "the" are intended to include the plural forms as well, unless the context clearly indicates otherwise.

As used herein, the terms "comprises," "comprising," "includes," "including," "has," "having," "contains", "containing," "characterized by" or any other variation thereof, are intended to cover a non-exclusive inclusion, subject to any limitation explicitly indicated. For example, a composition, mixture, process or method that comprises a list of elements is not necessarily limited to only those elements but may include other elements not expressly listed or inherent to such composition, mixture, process, or method.

The transitional phrase "consisting of' excludes any elements, steps, or ingredients not specified. If in the claim, such would close the claim to the inclusion of materials other than those recited except for impurities ordinarily associated therewith. When the phrase "consisting of" appears in a clause of the body of a claim, rather than immediately following the preamble, it limits only the element set forth in that clause; other elements are not excluded from the claim as a whole.

Where applicants have defined an invention or a portion thereof with an open-ended term such as "comprising," it should be readily understood that (unless otherwise stated) the description should be interpreted to also describe such an invention using the term "consisting of."

All numbers herein may be understood as modified by "about." As used herein, the term "about" is used to indicate that a value includes for example, the inherent variation of error for a measuring device, the method being employed to determine the value, or the variation that exists among the study subjects. Typically the term is meant to encompass approximately or less than 1%, 2%, 3%, 4%, 5%, 6%, 7%, 8%, 9%, 10%, 11%, 12%, 13%, 14%, 15%, 16%, 17%, 18%, 19% or 20% variability depending on the situation.

The use of the term "or" in the claims is used to mean "and/or" unless explicitly indicated to refer to alternatives only or the alternatives are mutually exclusive, although the disclosure supports a definition that refers to only alternatives and "and/or."

"Subject" as used herein refers to animals, including, for example, a mammalian subject diagnosed with or suspected of having or developing cancer(s). In an embodiment, the term "subject" can refer to a vertebrate having cancer or to a vertebrate deemed to be in need of cancer treatment. Subjects include warm-blooded animals, such as mammals, such as a primate, and, more preferably, a human. Non-human primates are subjects as well. The term subject includes domesticated animals, such as cats, dogs, apes, etc., livestock (for example, cattle, horses, pigs, sheep, goats, etc.) and laboratory animals (for example, mouse, rabbit, rat, gerbil, guinea pig, etc.). Thus, veterinary uses and medical formulations are contemplated herein.

"Administering" or "Administration" is referred to herein as providing a NK cell or a pharmaceutical composition of the present application to a subject. By way of example and not limitation, administration may be performed via parenteral, subcutaneous, intramuscular, intravenous, intra-articular, intrabronchial, intraabdominal, intracapsular, intracartilaginous, intracavitary, intracelial, intracerebellar, intracerebroventricular, intracolic, intracervical, intragastric, intrahepatic, intramyocardial, intraosteal, intrapelvic, intrapericardiac, intraperitoneal, intrapleural, intraprostatic, intrapulmonary, intrarectal, intrarenal, intraretinal, intraspinal, intrasynovial, intrathoracic, intrauterine, intravesical, bolus, vaginal, rectal, buccal, sublingual, intranasal, and transdermal. For example, injection may be performed by intravenous (*i.v.*) injection, sub-cutaneous (*s.c*.) injection, intradermal (*i.d*) injection, intraperitoneal (*i.p*.) injection, or intramuscular (*i.m*.) injection. One or more such routes may be employed. Parenteral administration can be, for example, by bolus injection or by gradual perfusion over time. Alternatively, or concurrently, administration may be by the oral route.

The use of the terms "treating," or "treatment" is referred to herein as administration of a NK cell or a pharmaceutical composition to a subject with the purpose to cure, alleviate, relieve, remedy, prevent, or ameliorate a disorder, symptoms of the disorder, a disease state secondary to the disorder, or predisposition toward the disorder. The terms "inhibiting," "reducing," or "prevention," or any variation of these terms, when used in the claims and/or the specification includes any measurable decrease or complete inhibition to achieve a desired result.

"Cancer(s)" that may be treated by the pharmaceutical composition of the application include those classified by site include cancer of the oral cavity and pharynx (lip, tongue, salivary gland, floor of mouth, gum and other mouth, nasopharynx, tonsil, oropharynx, hypopharynx, other oral/pharynx); cancers of the digestive system (esophagus; stomach; small intestine; colon and rectum; anus, anal canal, and anorectum; liver; intrahepatic bile duct; gallbladder; other biliary; pancreas; retroperitoneum; peritoneum, omentum, and mesentery; other digestive); cancers of the respiratory system (nasal cavity, middle ear, and sinuses; larynx; lung and bronchus; pleura; trachea, mediastinum, and other respiratory); cancers of the mesothelioma; bones and joints; and soft tissue, including heart; skin cancers, including melanomas and other non-epithelial skin cancers; Kaposi's sarcoma and breast cancer; cancer of the female genital system (cervix uteri; corpus uteri; uterus, ovary; vagina; vulva; and other female genital); cancers of the male genital system (prostate gland; testis; penis; and other male genital); cancers of the urinary system (urinary bladder; kidney and renal pelvis; ureter; and other urinary); cancers of the eye and orbit; cancers of the brain and nervous system (brain; and other nervous system); cancers of the endocrine system (thyroid gland and other endocrine, including thymus); lymphomas (Hodgkin's disease and non-Hodgkin's lymphoma), multiple myeloma, and leukemias (lymphocytic leukemia; myeloid leukemia; monocytic leukemia; and other leukemias).

Other cancers, classified by histological type, that may be suitable targets for the therapeutic compositions according to the present application include, but are not limited to, neoplasm, malignant; Carcinoma, not otherwise specified (NOS); Carcinoma, undifferentiated, NOS; Giant and spindle cell carcinoma; Small cell carcinoma, NOS; Papillary carcinoma, NOS; Squamous cell carcinoma, NOS; Lymphoepithelial carcinoma; Basal cell carcinoma, NOS; Pilomatrix carcinoma; Transitional cell carcinoma, NOS; Papillary transitional cell carcinoma; Adenocarcinoma, NOS; Gastrinoma, malignant; Cholangiocarcinoma; Hepatocellular carcinoma, NOS; Combined hepatocellular carcinoma and cholangiocarcinoma; Trabecular adenocarcinoma; Adenoid cystic carcinoma; Adenocarcinoma in adenomatous polyp; Adenocarcinoma, familial polyposis coli; Solid carcinoma, NOS; Carcinoid tumor, malignant; Bronchioloalveolar adenocarcinoma; Papillary adenocarcinoma, NOS; Chromophobe carcinoma; Acidophil carcinoma; Oxyphilic adenocarcinoma; Basophil carcinoma; Clear cell adenocarcinoma, NOS; Granular cell carcinoma; Follicular adenocarcinoma, NOS; Papillary and follicular adenocarcinoma; Nonencapsulating sclerosing carcinoma; Adrenal cortical carcinoma; Endometroid carcinoma; Skin appendage carcinoma; Apocrine adenocarcinoma; Sebaceous adenocarcinoma; Ceruminous adenocarcinoma; Mucoepidermoid carcinoma; Cystadenocarcinoma, NOS; Papillary cystadenocarcinoma, NOS; Papillary serous cystadenocarcinoma; Mucinous cystadenocarcinoma, NOS; Mucinous adenocarcinoma; Signet ring cell carcinoma; Infiltrating duct carcinoma; Medullary carcinoma, NOS; Lobular carcinoma; Inflammatory carcinoma; Paget's disease, mammary; Acinar cell carcinoma; Adenosquamous carcinoma; Adenocarcinoma w/ squamous metaplasia; Thymoma, malignant; Ovarian stromal tumor, malignant; Thecoma, malignant; Granulosa cell tumor, malignant; Androblastoma, malignant; Sertoli cell carcinoma; Leydig cell tumor, malignant; Lipid cell tumor, malignant; Paraganglioma, malignant; Extra-mammary paraganglioma, malignant; Pheochromocytoma; Glomangiosarcoma; Malignant melanoma, NOS; Amelanotic melanoma; Superficial spreading melanoma; Malig melanoma in giant pigmented nevus; Epithelioid cell melanoma; Blue nevus, malignant; Sarcoma, NOS; Fibrosarcoma, NOS; Fibrous histiocytoma, malignant; Myxosarcoma; Liposarcoma, NOS; Leiomyosarcoma, NOS; Rhabdomyosarcoma, NOS; Embryonal rhabdomyosarcoma; Alveolar rhabdomyosarcoma; Stromal sarcoma, NOS; Mixed tumor, malignant, NOS; Mullerian mixed tumor; Nephroblastoma; Hepatoblastoma; Carcinosarcoma, NOS; Mesenchymoma, malignant; Brenner tumor, malignant; Phyllodes tumor, malignant; Synovial sarcoma, NOS; Mesothelioma, malignant; Dysgerminoma; Embryonal carcinoma, NOS; Teratoma, malignant, NOS; Struma ovarii, malignant; Choriocarcinoma; Mesonephroma, malignant; Hemangiosarcoma; Hemangioendothelioma, malignant; Kaposi's sarcoma; Hemangiopericytoma, malignant; Lymphangiosarcoma; Osteosarcoma, NOS; Juxtacortical osteosarcoma; Chondrosarcoma, NOS; Chondroblastoma, malignant; Mesenchymal chondrosarcoma; Giant cell tumor of bone; Ewing's sarcoma; Odontogenic tumor, malignant; Ameloblastic odontosarcoma; Ameloblastoma, malignant; Ameloblastic fibrosarcoma; Pinealoma, malignant; Chordoma; Glioma, malignant; Ependymoma, NOS; Astrocytoma, NOS; Protoplasmic astrocytoma; Fibrillary astrocytoma; Astroblastoma; Glioblastoma, NOS; Oligodendroglioma, NOS; Oligodendroblastoma; Primitive neuroectodermal; Cerebellar sarcoma, NOS; Ganglioneuroblastoma; Neuroblastoma, NOS; Retinoblastoma, NOS; Olfactory neurogenic tumor; Meningioma, malignant; Neurofibrosarcoma; Neurilemmoma, malignant; Granular cell tumor, malignant; Malignant lymphoma, NOS; Hodgkin's disease, NOS; Hodgkin's; paragranuloma, NOS; Malignant lymphoma, small lymphocytic; Malignant lymphoma, large cell, diffuse; Malignant lymphoma, follicular, NOS; Mycosis fungoides; Other specified non-Hodgkin's lymphomas; Malignant histiocytosis; Multiple myeloma; Mast cell sarcoma; Immunoproliferative small intestinal disease; Leukemia, NOS; Lymphoid leukemia, NOS; Plasma cell leukemia; Erythroleukemia; Lymphosarcoma cell leukemia; Myeloid leukemia, NOS; Basophilic leukemia; Eosinophilic leukemia; Monocytic leukemia, NOS; Mast cell leukemia; Megakaryoblastic leukemia; Myeloid sarcoma; and Hairy cell leukemia.

An "effective amount," as used herein, refers to a dose of the modified NK cells or pharmaceutical composition that is sufficient to reduce the symptoms and signs of cancer, which include, but are not limited to, weight loss, pain or tumor mass which is detectable, either clinically as a palpable mass or radiologically through various imaging means.

In certain embodiments, it is desired to limit, reduce, or ameliorate the size of tumor or cancer lesions. The routes of administration will vary, naturally, with the location and nature of the lesion or site to be targeted, and include, *e.g*., regional, parenteral, intravenous, intramuscular, and/or systemic administration and formulation. Direct injection or injection into the vasculature or the vessels to and from and within an organ or tissue is specifically contemplated for target areas. Local, regional, or systemic administration also may be appropriate.

In the disclosure herein, the expression level or surface density of the cell surface antigen using FACS/flow cytometry analysis are defined in Table 1. The interpretation for the various expression levels in Table 1 is an example of defining the expression level of the cell surface antigen. It should be noted that the flow cytometry signal level intensity varies with the following factors: the flow cytometry, the software and different batches of antibody used.

**Table 1**

| Symbol | Interpretation |
|---|---|
| - | Flow cytometry signal level intensity less than or equal to 10⁰ (*i.e.,* 1) |
| + (Dim) | Flow cytometry signal level intensity between 10⁰ to 10¹ |
| + | Flow cytometry signal level intensity between 10¹ to 10² |
| + (hi) | Flow cytometry signal level intensity greater than or equal to 10² |

Unless otherwise defined, all technical and scientific terms used herein have the same meaning as commonly understood by one of ordinary skill in the art to which this invention belongs. All publications, patent applications, patents and other references cited herein are incorporated by reference in their entireties for the teachings relevant to the sentence and/or paragraph in which the reference is presented.

### Modified NK Cells and antigen-specific T cells

Naturally occurring or conventional NK cells have CD16⁺CD56⁺ phenotype. In one embodiment, the naturally occurring or conventional NK cells have CD3⁻CD14⁻CD19⁻ CD56⁺CD16⁺NKG2D⁺CD11c^{dim}phenotype, as illustrated in Table 2. Naturally occurring or conventional T cells have CD3⁺CD56⁺ phenotype. In one embodiment, the naturally occurring or conventional T cells have CD3⁺CD14⁻CD19⁻CD56⁺phenotype, as illustrated in Table 3.

In one embodiment, the present application provides a modified NK cell comprising a CD3⁻CD19⁻CD14⁻CD56^{hi}CD16^{dim}NKG2D⁺CD11c⁺CD86⁺HLA-DR⁺CD83⁻ cell phenotype, as illustrated in Table 2. The modified NK cell of the present application is non-naturally occurring. The modified NK cells comprise one or more of the fully activated dendritic (DC) cell surface antigens (for example, HLA-DR and CD86) and possess both NK cell function and DC cell function and an enhanced anti-cancer activity.

**Table 2. Phenotypic comparison of conventional NK cells and modified NK cells**

| | Conventional NK | Modified NK |
|---|---|---|
| CD3 | - | - |
| CD14 | - | - |
| CD19 | - | - |
| CD56 | + | hi |
| CD16 | + | dim |
| NKG2D | + | + |
| CD11c | dim | + |
| HLA-DR | - | + |
| CD86 | - | + |
| CD83 | - | - |

In one embodiment, the present application provides an antigen-specific T cell comprising a CD3⁺CD14⁻CD19⁻CD56⁺CD25^{hi}CD27⁻CD28⁻4-1BB⁺Perforin^{dim}Ganzyme B^{dim}CD107a⁺TNF-α⁺IFN-γ^{dim} cell phenotype, as illustrated in Table 3. The antigen-specific T cell of the present application is non-naturally occurring. The antigen-specific T cells comprise one or more of markers that do not express on the naturally occurring T cells, such as 4-1BB or CD25.

**Table 3. Phenotypic comparison of conventional T cells and antigen-specific T cells**

| | Conventional T | Antigen-specific T |
|---|---|---|
| CD3 | + | + |
| CD14 | - | - |
| CD19 | - | - |
| CD56 | + | + |
| TCRαβ | + | dim |
| TCRγδ | - | - |
| CD4 | - | - |
| CD8 | + | + |
| 4-1BB | - | + |
| CD27 | + | - |
| CD28 | + | - |
| CD25 | - | hi |
| CD69 | dim | dim |
| IFN-γ | dim | dim |
| TNF-α | + | + |
| Perforin | dim | dim |
| Granzyme B | + | dim |
| CD107a | dim | + |

Specifically, the present application provides a modified NK cell comprising a CD16^{dim}CD56^{hi} phenotype, wherein said CD16^{dim}CD56^{hi} NK cell does not comprise a CD83 cell surface antigen (having a phenotype of CD16^{di-}CD56^{hi}CD83⁻).

In one embodiment, the expression level or surface density of the cell surface antigen is quantified by exposing the modified NK cells or the antigen-specific T cells to a fluorescent dye-tagged specific anti-human monoclonal antibody (*e.g*., CD86-PE (Beckman Coulter; Cat. No: IM2729U) or Anti-human CD83-PE-Cy5 (BioLegend; Cat. No: 305310), followed by sorting of the modified NK cells or the antigen-specific T cells using flow cytometer (e.g. Navios, commercially available from Beckman Coulter, Inc., USA).

The modified NK cells or the antigen-specific T cells can be generated from a single individual, *e.g*. autologous or allogeneic.

### Pharmaceutical Composition

In one embodiment, the present application provides pharmaceutical compositions comprising a modified NK cell and antigen-specific T cells described herein, and a pharmaceutically acceptable carrier or excipient. In one embodiment, the modified NK cell and the antigen-specific T cells are manufactured together from the mononuclear cells, *e.g.* peripheral blood mononuclear cells (PBMCs).

The present application also provides methods of inhibiting cancer cells, and pharmaceutical compositions for use in such methods, by administering to a subject in need thereof the present pharmaceutical composition in an amount effective to inhibit cancer cells. Without being bound by any particular theory, it is believed that the present pharmaceutical composition inhibit cancer cells by the antigen-specific T cells and the modified NK cell with one or more of the NK cell/ DC cell functions: enhancing cytotoxicity, stimulating cancerspecific T lymphocyte proliferation or IFN-γ secretion.

Routes of administration of the present pharmaceutical compositions or modified NK cells include, but are not limited to, intravenous, intramuscular, subcutaneous, oral, topical, intradermal, transdermal, subdermal, parenteral, rectal, spinal, or epidermal administration. In one embodiment, the modified NK cells are administered by intravenous injection or infusion.

The pharmaceutical compositions of the present application can be prepared as injectables, either as liquid solutions or suspensions, or as solid forms which are suitable for solution or suspension in liquid vehicles prior to injection.

The present modified NK cells and the antigen-specific T cells are formulated into pharmaceutical compositions for delivery to a mammalian subject. The pharmaceutical composition is administered alone, and/or mixed with a pharmaceutically acceptable vehicle, excipient or carrier. Suitable vehicles are, for example, saline (*e.g.* normal saline), dextrose, glycerol, platelet-rich plasma (PRP), or the like, and combinations thereof. In addition, the vehicle can contain minor amounts of auxiliary substances such as wetting or emulsifying agents, pH buffering agents, or adjuvants. Pharmaceutically acceptable carriers can contain a physiologically acceptable compound that acts to, *e.g*., stabilize, or increase or decrease the absorption or clearance rates of the pharmaceutical compositions of the present application. Physiologically acceptable compounds can include, *e.g*., carbohydrates, such as glucose, sucrose, or dextran, antioxidants, such as ascorbic acid or glutathione, chelating agents, low molecular weight proteins, detergents, liposomal carriers, or excipients or other stabilizers and/or buffers. Other physiologically acceptable compounds include wetting agents, emulsifying agents, dispersing agents or preservatives. See *e.g.,* the 21st edition of Remington's Pharmaceutical Science, Mack Publishing Company, Easton, Pa. ("Remington's"). The pharmaceutical compositions of the present application can also include ancillary substances, such as pharmacological agents, cytokines, or other biological response modifiers.

Actual methods of preparing such dosage forms are known, or will be apparent, to those skilled in the art. See, *e.g*., Remington's Pharmaceutical Sciences, Mack Publishing Company, Easton, Pennsylvania, 21st edition.

The present pharmaceutical compositions can be administered in a single dose treatment or in multiple dose treatments on a schedule and over a time period appropriate to the age, weight and condition of the subject, the particular composition used, and the route of administration, whether the present pharmaceutical composition is used for prophylactic or curative purposes, etc. For example, in one embodiment, the pharmaceutical composition according to the present application is administered once per month, twice per month, three times per month, every other week (qow), once per week (qw), twice per week (biw), three times per week (tiw), four times per week (qiw), five times per week, six times per week, every other day (qod), daily (qd), twice a day (bid), three times a day (tid) or four times a day (qid).

The duration of treatment of the pharmaceutical composition according to the present application, *e.g*., the period of time over which the pharmaceutical composition is administered, can vary, depending on any of a variety of factors, *e.g*., subject response, etc. For example, the pharmaceutical composition can be administered over a period of time ranging from about one or more seconds to one or more minutes, one or more hours to one day to about one week, from about two weeks to about four weeks, from about one month to about two months, from about two months to about four months, from about four months to about six months, from about six months to about eight months, from about eight months to about 1 year, from about 1 year to about 2 years, or from about 2 years to about 4 years, or more.

It is advantageous to formulate parenteral pharmaceutical compositions in dosage unit form for ease of administration and uniformity of dosage. Dosage unit form as used herein refers to physically discrete units suited as unitary dosages for the subject to be treated; each unit containing a predetermined quantity of modified NK cells and the antigen-specific T cells calculated to produce the desired therapeutic effect in association with the required pharmaceutical carrier.

The data obtained from the cell culture assays and animal studies can be used in formulating a range of dosage for use in humans. In one embodiment, the dosage of such pharmaceutical compositions lies within a range of circulating concentrations that include the ED₅₀ with little or no toxicity. The dosage can vary within this range depending upon the dosage form employed and the route of administration utilized. In another embodiment, the therapeutically effective dose can be estimated initially from cell culture assays. A dose can be formulated in animal models to achieve a circulating plasma concentration range that includes the IC₅₀ (*i.e.,* the concentration of the modified NK cells and the antigen-specific T cells which achieves a half-maximal inhibition of symptoms) as determined in cell culture. Sonderstrup, Springer, Sem. Immunopathol. 25: 35-45, 2003. Nikula et al, Inhal. Toxicol. 4(12j: 123- 53, 2000.

The pharmaceutical composition is formulated to contain an effective amount of the modified NK cells and the antigen-specific T cells, wherein the amount depends on the animal to be treated and the condition to be treated. The specific dose level for any particular subject depends upon a variety of factors including the activity of the specific modified NK cells and the antigen-specific T cells, the age, body weight, general health, sex, diet, time of administration, route of administration, and rate of excretion, drug combination and the severity of the particular disease undergoing therapy. An exemplary, non-limiting range for a therapeutically or prophylactically effective amount of the pharmaceutical compositions of the present application is at least about l × 10³ cells per dose to about l × 10⁹ cells per dose. Other dosages are also possible, including, but not limited to, 1 × 10⁴, 1 × 10⁵, 1 × 10⁶, 1 × 10⁷, 1 × 10⁸ or 1 × 10⁹ cells per dose.

The pharmaceutical composition can be administered alone or in combination with another therapeutic agent, *e.g*., chemotherapy, radiotherapy or targeted therapy or cancer vaccine.

### Methods of Manufacturing the Pharmaceutical Compositions

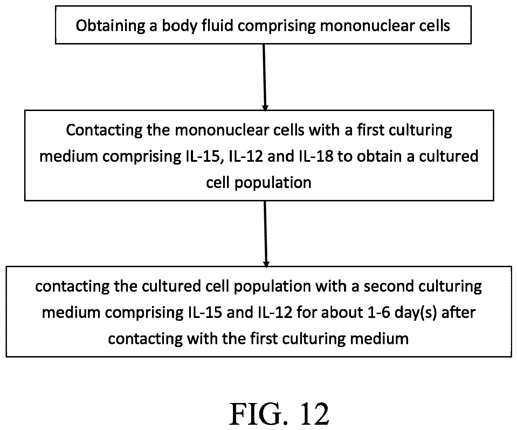

In one embodiment, the methods of manufacturing the modified NK cells and the antigen-specific T cells are illustrated as in FIG. 12. Briefly, the method comprises at least the steps of: obtaining mononuclear cells; contacting the mononuclear cells with a first culturing medium comprising IL-15, IL-12 and IL-18 for about 1-6 day(s) to obtain a cultured cell population; and contacting the cultured cell population with a second culturing medium comprising IL-15 and IL-12 for about 1-6 day(s) after contacting with the first culturing medium; wherein a negative selection of CD3⁻CD19⁻CD14⁻ is not performed on the mononuclear cells prior to the contact step..

Preferably, the mononuclear cells used herein for the selection or production of the modified NK cells and antigen-specific T cell are mononuclear cells, on which a negative selection of CD3⁻CD14⁻CD19⁻ cells are not performed, as the initial cell population for the expansion culture.

One embodiment of the disclosure provides methods for identifying an initial cell from a sample, comprising no steps of depleting one or more of the following cell surface antigens from a mononuclear cell in said sample: CD3, CD14, or CD19.

The culturing time of the composition comprising IL-12 may be critical for function of the modified NK cells. In an embodiment, mononuclear cells may be cultured with IL-12, *e.g.* human IL-12, for 1 to 12 days, preferably 1, 2, 3, 4, 5, 6, 7, 8, 9, 10, 11, or 12 days, to generate the modified NK cells. In an embodiment, mononuclear cells cultured with IL-12 for 3, 6, 9 or 12 days generate modified NK cells with similar cell yields and phenotypic patterns. In another embodiment, prolong exposure of IL-12, *e.g.* 12 days, augmented cytotoxicity and antigen-presenting cell activities of the cultured cells as compared to the cells with IL-12 exposure for less time, *e.g.* 9 days.

In one embodiment, the composition for culturing the cells further includes IL-18. In one embodiment, the effective concentration of IL-18 is about 1 to 300 ng/mL, such as, for example, 50, 100, 150, 200, 250, 300 ng/mL. In another embodiment, the effective concentration of IL-18 is about 10 to about 250 ng/mL, or any value or range of values there between in 10 ng/mL increments (*e.g.*, about 30 ng/mL, about 220 ng/mL, etc.).

In another embodiment, IL-18 shed effect on the phenotypic patterns and the functional activities of the modified NK cells. Long term IL-18 exposure may have the opposite effects on cell size, phenotype and functional activities. In an embodiment, mononuclear cells may be cultured with IL-18, *e.g.* human IL-18, for 1 to 12 days, preferably 1, 2, 3, 4, 5, 6, 7, 8, 9, 10, 11, or 12 days, to generate the modified NK cells. In an embodiment, mononuclear cells cultured with IL-18 for 3, 6, 9 or 12 days generate modified NK cells with various phenotypes. In another embodiment, adequate exposure of IL-18, *e.g.* 6 days, may have opposite effects on cell size, phenotype and function of the modified NK cells as compared to the cells with IL-18 exposure for less time, *e.g.* 6 days.

In one embodiment, a mononuclear cells are in contact with a culture composition comprising IL-18, IL-15 and/or IL-12. In another embodiment, the mononuclear cells are mixed with a composition substantially consisting of hematopoietic cell medium (*e.g.* X-vivo 20), IL-18, IL-15, IL-12 and serum protein (*e.g.* human platelet lysate). In still another embodiment, the mononuclear cells are mixed with a composition substantially consisting of X-vivo 20, IL-18, IL-15, IL-12 and human platelet lysate.

In another embodiment, the composition further comprises a hematopoietic cell medium. Non limiting example of the hematopoietic cell medium includes X-vivo 10, X-vivo 15, X-vivo 20 (commercially available from Lonza, Switzerland) and AIM-V (commercially available from ThermoFisher Scientific, United States).

In yet another embodiment, the composition further comprises serum protein, *e.g.* human platelet lysate. In the present application, "serum protein" are the proteins present in blood or plasma that serve many different functions, including transport and regulation of a cellular activity. Non limiting example of the serum protein includes enzymes, complement components, protease inhibitors, kinin precursors, serum albumin, globulins, and fibrinogen etc.

Non limiting example of the composition for culturing the cells includes (a) hematopoietic cell medium + IL-15 + IL-18; (b) hematopoietic cell medium + IL-12 + IL-18; (c) hematopoietic cell medium + IL-15 + IL-12 + IL-18; (d) X-vivo 20 + IL-15 + IL-18; (e) X-vivo 20 + IL-12 + IL-18; (f) X-vivo 20 + IL-15 + IL-12 + IL-18; (g) AIM-V + IL-15 + IL-18; (h) AIM-V + IL-12 + IL-18; (i) AIM-V + IL-15 + IL-12 + IL-18; (j) hematopoietic cell medium + IL-15 + IL-18 + serum protein; (k) hematopoietic cell medium + IL-12 + IL-18 + serum protein; (l) hematopoietic cell medium + IL-15 + IL-12 + IL-18 + serum protein; (m) X-vivo 20 + IL-15 + IL-18 + serum protein; (n) X-vivo 20 + IL-12 + IL-18 + serum protein; (o) X-vivo 20 + IL-15 + IL-12 + IL-18 + serum protein; (p) AIM-V + IL-15 + IL-18 + serum protein; (q) AIM-V + IL-12 + IL-18 + serum protein; and (r) AIM-V + IL-15 + IL-12 + IL-18 + serum protein.

In one embodiment, the culturing mediums are used for culturing the modified NK cells and the antigen-specific T cell in the presence of 5% CO₂ at 37 °C.

The methods according to some embodiments of the application, which do not include a negative selection of CD3⁻CD14⁻CD19⁻, enhances the yield of the modified NK cells and antigen-specific T cells. Yield of the modified NK cells was determined by the purity of mononuclear cells via FACS and viable counts. Other assays for cell proliferation are well known in the art, *e.g*., clonogenic assays, metabolic assays, and direct proliferation assays.

An exemplary non-limiting range for the contact time of themononuclear cells and the culturing mediums is from about 1 minute to about 1 hour, from about 1 hour to about 24 hours, from about 1 day to about 3 days, from about 1 day to about 6 days, from about 1 day to about 9 days, from about 1 day to about 12 days, from about 3 days to about 6 days, from about 3 days to about 9 days, from about 3 days to about 12 days, from about 6 days to about 9 days, from about 6 days to about 12 days, or at least 1 day. In one embodiment, the contact time is about 3 days. In another embodiment, the contact time is about 6 days.

In one embodiment, the mononuclear cells are in contact with a first culturing medium comprising an IL-15, an IL-12 and an IL-18; followed by contacting with a second culturing medium comprising an IL-15 and an IL-12. In another embodiment, the first and second culturing mediums further comprise a hematopoietic cell medium, such as AIM-V or X-vivo, and/or a serum protein, such as human platelet lysate.

In one embodiment, the modified NK cell and antigen-specific T cell may be manufactured by the following steps:
(a) Contacting l×10⁶/mL mononuclear cells with a composition comprising AIM-V medium, HPL (concentration: 4%w/w), IL-15 (concentration: 30 ng/mL), IL-12 (concentration: 3 ng/mL) and IL-18 (concentration: 50 ng/mL) on day 0.
(b) Harvesting and spinning down half of the initial cells in step (a), followed by resuspending cell pellet with a composition comprising AIM-V medium, HPL, IL-15 and IL-12 on day 6.
(c) Collecting all of the cultured cells in step (b) on day 12.

Optionally, the composition for culturing the cells may be replaced with a fresh medium, with the same constituents on day 0 and day 6, respectively, on day 3 and day 9. for example, the initial cell may be cultured as follows:
(a) Contacting l×10⁶/mL mononuclear cells with a composition comprising AIM-V medium, HPL (concentration: 4%w/w), IL-15 (concentration: 30 ng/mL), IL-12 (concentration: 3 ng/mL) and IL-18 (concentration: 50 ng/mL) on day 0.
(b) Replacing a medium with the composition comprising AIM-V medium, HPL, IL-15, IL-12 and IL-18 on day 3.
(c) Harvesting and spinning down half of the cultured cells in step (b), followed by resuspending cell pellet with a composition comprising AIM-V medium, 4%w/w HPL, 30 ng/mL of IL-15 and 3 ng/mL of IL-12 on day 6.
(d) Replacing a medium with the composition comprising AIM-V medium, HPL, IL-15, and IL-12 on day 9.
(e) Collecting all of the cultured cells in step (d) on day 12.

Alternatively, the composition for culturing the cells may be replaced with a fresh medium, with the same constituents on day 0 and day 6, respectively, on day 3 and day 9. for example, the initial cell may be cultured as follows:
(a) Contacting l×10⁶/mL mononuclear cells with a composition comprising AIM-V medium, HPL (concentration: 4%w/w), IL-15 (concentration: 30 ng/mL), IL-12 (concentration: 3 ng/mL) and IL-18 (concentration: 50 ng/mL) on day 0.
(b) Replacing a medium with the composition comprising AIM-V medium, HPL, IL-15, IL-12 and IL-18 on day 3.
(c) Harvesting and spinning down half of the cultured cells in step (b), followed by resuspending cell pellet with a composition comprising AIM-V medium, 4%w/w HPL, 30 ng/mL of IL-15, 3 ng/mL of IL-12 and 50 ng/mL of IL-18 on day 6.
(d) Replacing a medium with the composition comprising AIM-V medium, HPL, IL-15, IL-12 and IL-18 on day 9.
(e) Collecting all of the cultured cells in step (d) on day 12.

The following examples and embodiments of specific aspects for carrying out the present invention are offered for illustrative purposes only, and are not intended to limit the scope of the present invention in any way.

### EXAMPLES

### Example 1: Preparation of initial cell

40 mL of peripheral blood was collected from a healthy volunteer into vacuum tubes containing K2EDTA. The blood sample was mixed with equal volume of pre-warmed phosphate-buffered saline (PBS) (Biological Industries, Israel). The 40 mL diluted peripheral blood aliquot was placed into a 50 mL centrifuge tube and loaded 10 mL pre-warmed Ficoll-Paque^{™} PREMIUM. The centrifuge tube was centrifuged at 2000 rpm, in room temperature for 30 min. The mononuclear cells in the interface layer were collected and washed once in PBS. The cell pellet was re-suspended into a density of 10⁶ cells/100 mL MACS buffer.

### Example 2: Culture of modified NK cell and antigen-specific T cell

The mononuclear cell from Example 1 was cultured in AIM-V medium which contains 4% HPL, hIL-15 (day 0, 3, 6, 9), 3ng/ml hIL-12 (day 0, 3, 6, 9) and hIL-18 (day 0, 3, 6) for 12 days. Total cell count and viability were determined by trypan blue dye exclusion. The following formula calculated the absolute cell number.

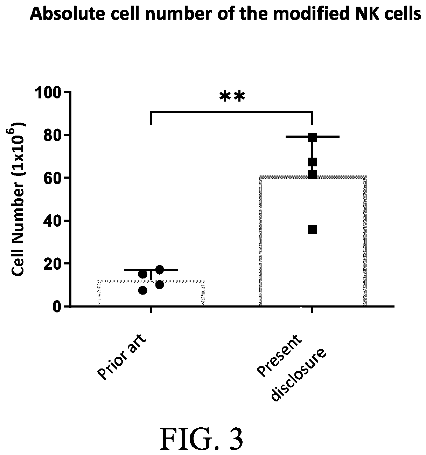

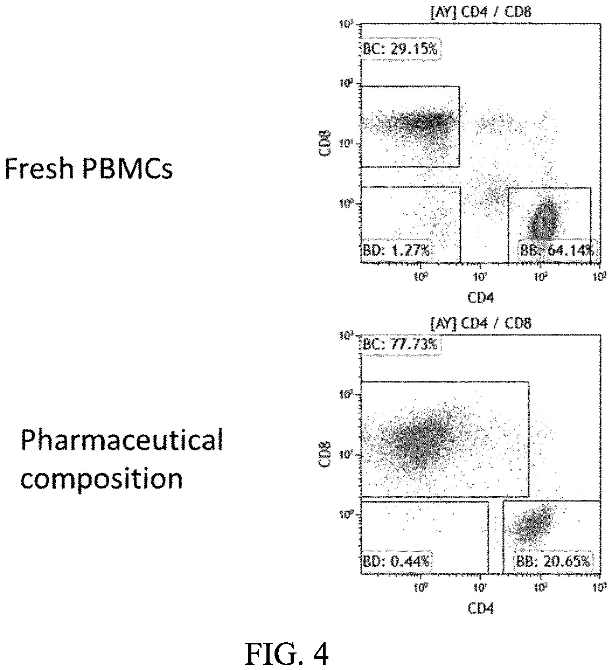

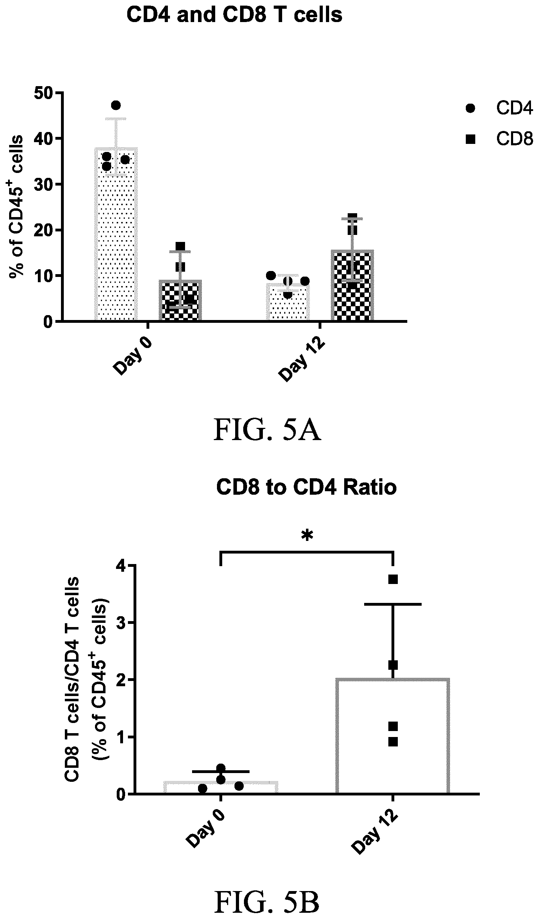

Results: As shown in Table 3 and FIG. 3, the enrichment step in the prior art significantly reduced the number of the initial cells for the manufacture. Although the purity of the modified NK cells is higher, the absolute cell number of the modified NK cell of the prior art is only one-fifth of that of the modified NK cell of the present disclosure. On the other hand, with the method of the present disclosure, the manufacture significantly expanded CD8 T cell population, as shown in FIGs. 4, 5A and 5B.

**Table 4. The purity and cell number of the obtained modified NK cells**

| | **Initial Cell Enrichment** | | | |
|---|---|---|---|---|
| Manufacture Protocol | Input Cells^{#1} (x10⁶) | Purity of CD3⁻CD14⁻ CD19⁻Cells (%) | Efficacy of Cell Enrichment (%) | |
| Prior art (n=4) | 100.00 | 18.19 | 104.54 | |
| Present disclosure (n=4) | - | - | - | |
| | | | | |

| | **Manufacture** | | | |
|---|---|---|---|---|
| Manufacture Protocol | Initial cel (x10⁶) | Cell expansion rate (%) | Purity of modified NK cell^{#2} (%) | The Absolute Cell Number of modified NK cell^{†} (x10⁶) |
| Prior art (n=4) | 18.24 | 88.2 | 76.63 | 12.57 |
| Present disclosure (n=4) | 100.00 | 164.00 | 38.87 | 61.02 |

| | | | | |
|---|---|---|---|---|
| ^{#1}: Input cells are 100 × 10⁶ as an example ^{#2}: The purity is defined as the percentage of CD3⁻CD14⁻CD19⁻CD56⁺HLA-DR⁺CD86⁺ cells ^{†} Calculation of Absolute Cell Number: Prior art manufacture: 100 × 10⁶ PBMCs (Input cells) x the purity of the CD3⁻CD14⁻CD19⁻ cells x the efficacy of cell enrichment (depletion of CD3⁺CD14⁺CD19⁺ cells) x cell expansion rate x purity (CD3⁻CD14⁻CD19⁻CD56⁺HLA-DR⁺CD86⁺); Present disclosure manufacture: 100 ×10⁶ PBMCs (Initial cells) x cell expansion rate x purity (CD3⁺CD14⁺CD19⁺CD56⁺HLA-DR⁺CD86⁺). Statistical analyses were performed using Student's t-test and results were considered significant if P<0.05. ns: not significant. *: P<0.05, **: P<0.01, ***: P<0.001. | | | | |

### Example 3: Ag-Specific CD8 T cells

PBMCs were cultured in AIM-V medium which containing 4% HPL, 30ng/ml hIL-15 (day 0, 3, 6, 9), 3 ng/ml hII,-12 (day 0, 3, 6, 9) and 50 ng/ml hII,-18 (day 0, 3, 6) for 12 days. the manufacture was stained with mAbs against CD45-ECD, CD56-APC-Alexa Flour 700, CD3-APC-Alexa Flour 750, CD14-APC-Alexa Flour 750, CD19-APC-Alexa Flour 750, CD8-Krome Orange (Beckman Coulter), TCRαβ-FITC, 4-1BB-PE, CD25-PE, CD28-PerCp-Cy5.5, CD4-PE-Cy7, TCRγδ-APC, CD27-Pacific Blue (Biolegend). Samples acquisition via Navios Flow Cytometer and data analysis via Kaluza software (Beckman Coulter). Statistical analyses were performed using Student's *t*-test and results were considered significant if P<0.05. ns: not significant. *: P<0.05, **: P<0.01, ***: P<0.001.

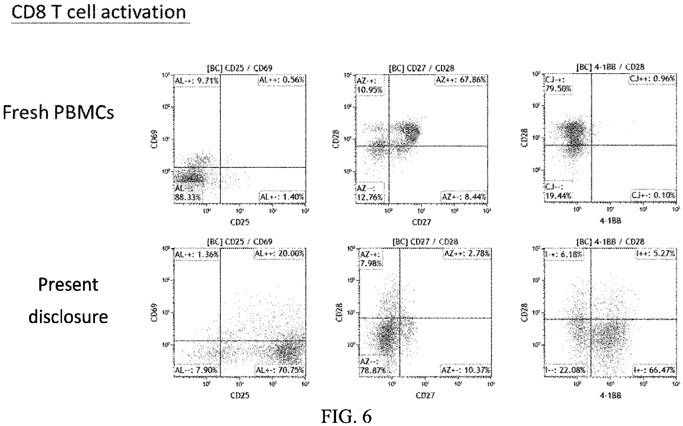

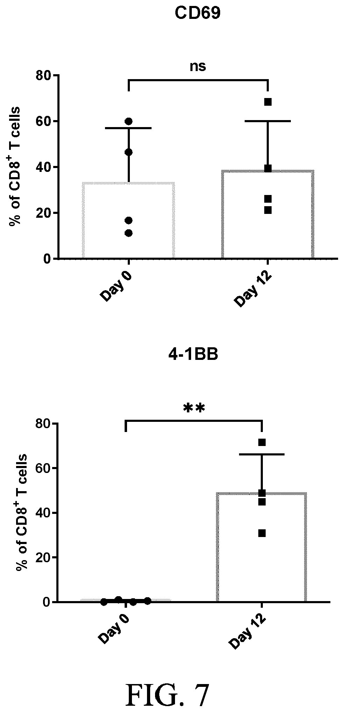

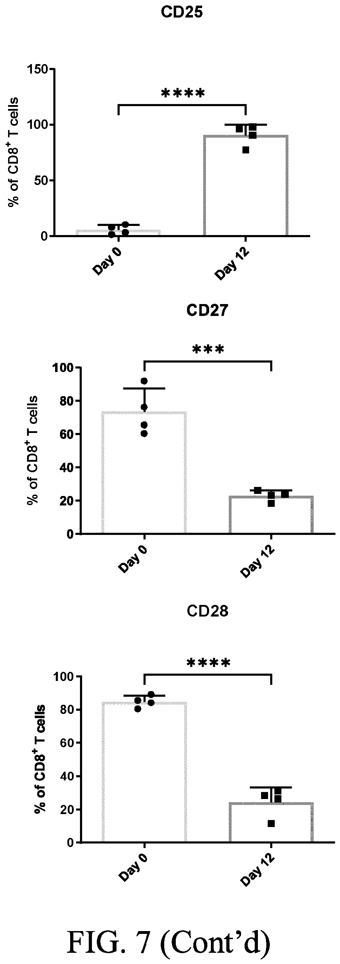

Results: Activation-induced markers (AIMs), including up-regulation of CD25 (PLoS ONE, 2017, DOI: 10.1371/journal.pone.0186998), CD69, 4-1BB (CD137), IFN-γ (Sig Transduct Target Ther, 2021, DOI: 10.1038/s41392-021-00589-1), or down-regulation of CD27 and CD28 (Med Microbiol Immunol, 2019, DOI: 10.1007/s00430-019-00608-7), are used in antigen-specific T cell identification. As shown in FIGs. 6 and 7, in the expanded CD8 T cell population of the manufacture described in the present disclosure, CD8 T cell expressed Ag-specific T cell phenotype such as up-regulation of activation-induced markers 4-1BB and CD25, and down-regulation of activation-induced markers CD27 and CD28, which indicated CD8 T cell activation. The expression of the AIMs is about 4 to 170-fold increase comparing to the fresh T cells in the PBMC.

### Example 4: Degranulation of CD8 T cells

PBMCs were cultured in AIM-V medium which containing 4% HPL, 30ng/ml hIL-15 (day 0, 3, 6, 9), 3ng/ml hIL-12 (day 0, 3, 6, 9) and 50ng/ml hIL-18 (day 0, 3, 6) for 12 days. The manufacture of the present disclosure was stained with mAbs against CD56-APC-Alexa Flour 700, CD14-APC-Alexa Flour 750, CD19-APC-Alexa Flour 750 (Beckman Coulter), Perforin-FITC, 4-1BB-PE, CD107a-PE/Dazzle 594, IFN-γ-PerCp-Cy5.5, TNF-α-PE-Cy7, Granzyme B-Pacific Blue, CD3-BV510 (Biolegend). Samples acquisition via Navios Flow Cytometer and data analysis via Kaluza software (Beckman Coulter). Statistical analyses were performed using Student's *t*-test and results were considered significant if P<0.05. ns: not significant. *: P<0.05, **: P<0.01, ***: P<0.001.

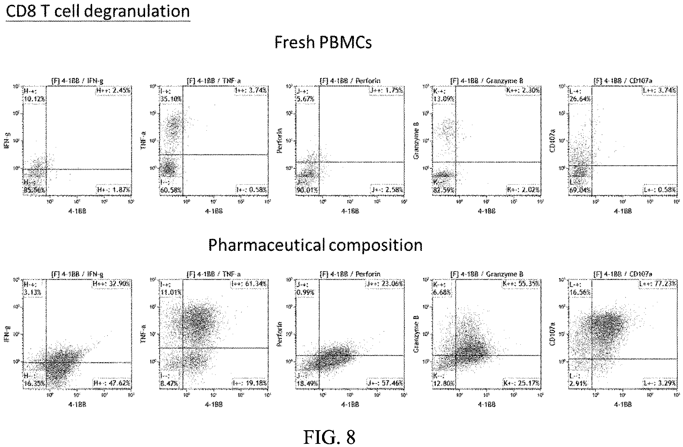

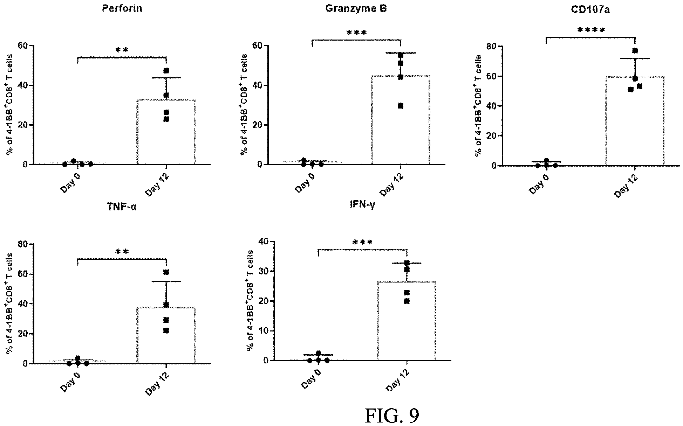

Results: As shown in FIGs. 8 and 9, in the manufacture described in the present disclosure, the CD8 T cells were induced to deliver degranulation molecules, such as Perforin, Granzyme B, CD107a, TNF-α and IFN-γ, which is about 60-fold increase comparing to the fresh T cells in the PBMC.

### Example 5: Pharmaceutical Compositions Executed Anti-Tumor Activities

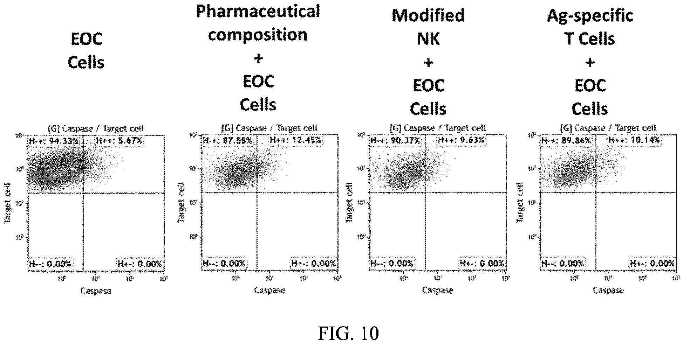

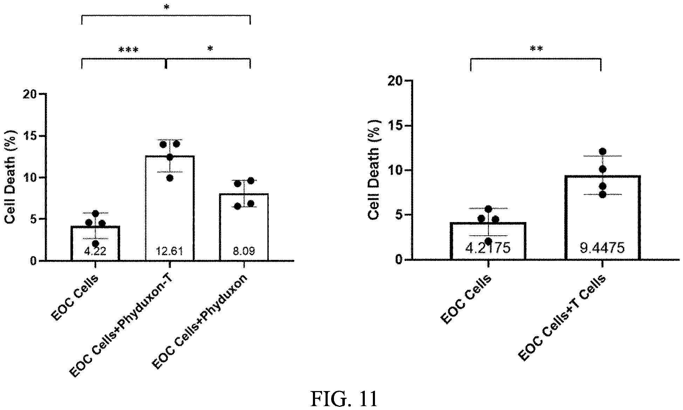

The modified NK cells and the antigen-specific T cells were sorted out from the manufacture by depleting CD3⁺ cells and CD56⁺ cells via magnetic beads, respectively. Evaluation of cytotoxicity of the modified NK cells and the antigen-specific T cells was performed by PanToxilux kit (OncoImmunin, Inc.). Human epithelial ovarian cancer (EOC) cells served as target cells and were stained with TFL4 under the optimal concentration for 50 minutes. Co-incubated TFL-4 labeled target cells and the modified NK cells or the antigen-specific T cells with the caspase substrate under 37°C for 60 minutes. Harvested the cells and analyzed the cell death by determining the signal of TFL-4⁺substrate⁺ via flow cytometry. Statistical analyses were performed using Student's t-test and results were considered significant if P<0.05. ns: not significant. *: P<0.05, **: P<0.01, ***: P<0.001
Results: As shown in FIGs. 10 and 11, the percentage of caspase positive target cells (with only modified NK cells or Ag-specific T cells) was 9.63% and 10.14%, respectively, whereas the percentage of caspase positive target cells (with the pharmaceutical composition of the present disclosure) was 12.45%, which is at least 22.7% increase comparing to modified NK cells alone or Ag-specific T cells alone. This result shows the pharmaceutical composition of the present disclosure, which was generated from the patient, exhibited tumoricidal activity against primary tumor cells from the same patient.

**Table 5. Reagents and kits used in the present disclosure**

| Name | Cat. # | Vender |
|---|---|---|
| **Cultivation** | | |
| Phosphate-Buffered Saline, 1 X without Ca²⁺ and Mg²⁺ | 21-040-CV | Corning |
| Ficoll- paque Premium 1.077 | 17-5442-03 | Cytiva |
| TM CTS AIM V Serum Free Medium | 0870112DK | Gibco |
| TM GMP UltraGRO -Advanced (HPL) | HPCFDCGL10 | AventaCell |
| Recombinant Human IL-15 GMP | 247-GMP-025 | R&D SYSTEMS |
| MACS^{®} GMP Recombinant Human IL-12 | 170-076-174 | Miltenyi Biotec |
| Recombinant Human IL-18 (carrier-free) | 592106 | BioLegend |

| **Functional assay** | | |
|---|---|---|
| Killing assay Kit (PanToxiLux^{™}) | PTL8028 | OncoImmunin |
| Collagenase Type IV | C5138 | Sigma-Aldrich |
| Dnase I | D5025 | Sigma-Aldrich |
| Hyaluronidase | H3757 | Sigma-Aldrich |
| Medium 199, Earle's Salts | 11150059 | Gibco |
| MCDB 105 Medium | 117-500 | Cell Applications |
| CellTrace^{™} CFSE Cell Proliferation Kit | C34554 | Invitrogen |
| CD25 Biotin | 302624 | BioLegend |
| Streptavidin Microbeads | 130-048-101 | Miltenyi Biotec |
| CliniMACS PBS/EDFABuffer | 700-25 | Miltenyi Biotec |
| Ultra-LEAF^{™} Purified anti-human CD3 Antibody | 317326 | BioLegend |
| Ultra-LEAF^{™} Purified anti-human CD28 Antibody | 302934 | BioLegend |
| Recombinant Human IL-2 (carrier-free) | 791908 | BioLegend |
| Recombinant Human IL-15 (carrier-free) | 570308 | BioLegend |
| CD3 APC-Alexa Flour 750 | A66329 | Beckman Coulter |
| CD4 APC | 317416 | BioLegend |
| CD86 PE/Cy7 | 305422 | BioLegend |
| CD8a Pacific Blue | 301023 | BioLegend |

| **Phenotype** | | |
|---|---|---|
| CD45 ECD | A07784 | Beckman Coulter |
| CD3 APC-Alexa Flour 750 | A66329 | Beckman Coulter |
| CD14 APC-Alexa Flour 750 | A86052 | Beckman Coulter |
| CD19 APC-Alexa Flour 750 | A78838 | Beckman Coulter |
| CD56 APC-Alexa Flour 700 | B10822 | Beckman Coulter |
| CD16 PE/Cy7 | 6607118 | Beckman Coulter |
| NKG2D(CD314) PE | A08934 | Beckman Coulter |
| CD11c APC | 301614 | BioLegend |
| HLA-A,B,C Pacific Blue | 311418 | BioLegend |
| HLA-DR Krome Orange | B00070 | Beckman Coulter |
| CD86 Alexa Fluor 488 | 305414 | BioLegend |
| CD83 PE/Cy5 | 305310 | BioLegend |
| TCR α/β FITC | 306706 | BioLegend |
| TCR γ/δ APC | 331212 | BioLegend |
| CD3 Brilliant Violet 510 | 300448 | BioLegend |
| CD4 PE/Cy7 | 6607101 | Beckman Coulter |
| CD8a APC | 301014 | BioLegend |
| CD8 Krome Orange | B00067 | Beckman Coulter |
| 4-1BB(CD137) PE | 309804 | BioLegend |
| CD27 Pacific Blue | 356414 | BioLegend |
| CD28 PerCP/Cyanine5.5 | 302922 | BioLegend |
| CD25 PE | 302606 | BioLegend |
| CD69 Pacific Blue | 310920 | BioLegend |
| PD-1 (CD279) PerCP/Cyanine5.5 | 329914 | BioLegend |
| CD107a PE/Dazzle^{™} 594 | 328646 | BioLegend |
| Perforin FITC | 308104 | BioLegend |
| Granzyme B Pacific Blue | 515408 | BioLegend |
| IFN-γ PerCP/Cyanine5.5 | 502526 | BioLegend |
| TNF-α PE/Cy7 | 502930 | BioLegend |
| Mouse IgG1 PE | IM0670U | Beckman Coulter |
| Mouse IgG1 PE/Cy7 | 6607099 | Beckman Coulter |
| Mouse IgG1 Krome Orange | A96415 | Beckman Coulter |
| Mouse IgG2b, κ Isotype Ctrl Alexa Fluor 488 | 400329 | BioLegend |
| Mouse IgG2b, κ Isotype Ctrl FITC | 400310 | BioLegend |
| Mouse IgG1, κ Isotype Ctrl PE | 400112 | BioLegend |
| Mouse IgG1, κ Isotype Ctrl PerCP/Cyanine5. 5 | 400150 | BioLegend |
| Mouse IgG1, κ Isotype Ctrl PE/Cy5 | 400116 | BioLegend |
| Mouse IgG1, κ Isotype Ctrl PE/Cy7 | 400125 | BioLegend |
| Mouse IgG1, κ Isotype Ctrl APC | 400122 | BioLegend |
| Mouse IgG1, κ Isotype Ctrl Pacific Blue | 400131 | BioLegend |

While specific aspects of the invention have been described and illustrated, such aspects should be considered illustrative of the invention only and not as limiting the invention as construed in accordance with the accompanying claims. All publications and patent applications cited in this specification are herein incorporated by reference in their entirety for all purposes as if each individual publication or patent application were specifically and individually indicated to be incorporated by reference for all purposes. Although the foregoing invention has been described in some detail by way of illustration and example for purposes of clarity of understanding, it will be readily apparent to one of ordinary skill in the art in light of the teachings of this invention that certain changes and modifications can be made thereto without departing from the spirit or scope of the appended claims.

Without further elaboration, it is believed that one skilled in the art can, based on the above description, utilize the present invention to its fullest extent. The following specific examples are, therefore, to be construed as merely illustrative, and not limitative of the remainder of the disclosure in any way whatsoever. All publications cited herein are incorporated by reference.

The invention is now described and further exemplified by the following embodiments:
Embodiment 1. A method of manufacturing pharmaceutical composition for treating cancer, comprising
   obtaining mononuclear cells from a subject with cancer;
   contacting the mononuclear cells with a first culturing medium comprising IL-15, IL-12 and IL-18 for about 1-6 day(s) to obtain a cultured cell population; and
   contacting the cultured cell population with a second culturing medium comprising IL-15 and IL-12 for about 1-6 day(s) after contacting with the first culturing medium;
   wherein a negative selection of CD3⁻CD19⁻CD14⁻ is not performed on the mononuclear cells prior to the contact step.
Embodiment 2. The method of embodiment 1, wherein the mononuclear cells are derived from peripheral blood, cord blood or bone marrow.
Embodiment 3. The method of embodiment 1 or 2, wherein the first culturing medium and the second culturing medium further comprises a hematopoietic cell medium.
Embodiment 4. The method of embodiment 3, wherein the hematopoietic cell medium comprises AIM-V medium.
Embodiment 5. The method of embodiment 3 or 4, wherein the first culturing medium further comprises a serum protein.
Embodiment 6. The method of embodiment 5, wherein the serum protein comprises a human platelet lysate.
Embodiment 7. A pharmaceutical composition for treating cancer, comprising
   (a) a modified nature killer (NK) cell with a phenotype of CD3⁻CD19⁻CD14⁻ CD56^{hi}CD 16^{dim}NKG2D⁺CD11c⁺CD86⁺HLA-DR⁺CD83⁻;
   (b) an antigen-specific T cell with a phenotype of 4-1BB⁺CD8⁺; and
   (c) a pharmaceutically acceptable carrier or excipient.
Embodiment 8. The pharmaceutical composition of embodiment 7, wherein the NK cell and the antigen-specific T cell are generated by any one of the method of embodiments 1-6.
Embodiment 9. A method of treating cancer, comprising
   administering an effective amount of the pharmaceutical composition of embodiment 7 or 8 to a subject in need thereof.
Embodiment 10. The method of embodiment 9, wherein the effective amount is about l×10³ to about l×10⁹ cells per dose.
Embodiment 11. The method of embodiment 9 or 10, wherein the modified NK cell or the antigen-specific T cell is autologous or allogeneic.
Embodiment 12. The method of embodiment 9, 10 or 11 wherein the modified NK cell or the antigen-specific T cell is derived from peripheral blood, cord blood or bone marrow.
Embodiment 13. The method of embodiment 9, 10, 11 or 12 wherein the cancer comprises cancer of the oral cavity and pharynx (lip, tongue, salivary gland, floor of mouth, gum and other mouth, nasopharynx, tonsil, oropharynx, hypopharynx, other oral/pharynx); cancers of the digestive system (esophagus; stomach; small intestine; colon and rectum; anus, anal canal, and anorectum; liver; intrahepatic bile duct; gallbladder; other biliary; pancreas; retroperitoneum; peritoneum, omentum, and mesentery; other digestive); cancers of the respiratory system (nasal cavity, middle ear, and sinuses; larynx; lung and bronchus; pleura; trachea, mediastinum, and other respiratory); cancers of the mesothelioma; bones and joints; and soft tissue, including heart; skin cancers, including melanomas and other non-epithelial skin cancers; Kaposi's sarcoma and breast cancer; cancer of the female genital system (cervix uteri; corpus uteri; uterus, ovary; vagina; vulva; and other female genital); cancers of the male genital system (prostate gland; testis; penis; and other male genital); cancers of the urinary system (urinary bladder; kidney and renal pelvis; ureter; and other urinary); cancers of the eye and orbit; cancers of the brain and nervous system (brain; and other nervous system); cancers of the endocrine system (thyroid gland and other endocrine, including thymus); lymphomas (Hodgkin's disease and non-Hodgkin's lymphoma), multiple myeloma, and/or leukemias (lymphocytic leukemia; myeloid leukemia; monocytic leukemia; and other leukemias).
Embodiment 14. A pharmaceutical composition according to any of embodiments 7 and 8, for use in a method of treating cancer, said method comprising
   Administering an effective amount of the pharmaceutical composition of embodiment 7 or 8 to a subject in need thereof.
Embodiment 15. The pharmaceutical composition for use of embodiment 14, wherein the effective amount is about l×10³ to about l×10⁹ cells per dose.
Embodiment 16. The pharmaceutical composition for use of embodiment 14 or 15, wherein the modified NK cell or the antigen-specific T cell is autologous or allogeneic.
Embodiment17. The pharmaceutical composition for use of embodiment 14, 15 or 16, wherein the modified NK cell or the antigen-specific T cell is derived from peripheral blood, cord blood or bone marrow.
Embodiment 18. The pharmaceutical composition for use of embodiment 14, 15, 16 or 17, wherein the cancer comprises cancer of the oral cavity and pharynx (lip, tongue, salivary gland, floor of mouth, gum and other mouth, nasopharynx, tonsil, oropharynx, hypopharynx, other oral/pharynx); cancers of the digestive system (esophagus; stomach; small intestine; colon and rectum; anus, anal canal, and anorectum; liver; intrahepatic bile duct; gallbladder; other biliary; pancreas; retroperitoneum; peritoneum, omentum, and mesentery; other digestive); cancers of the respiratory system (nasal cavity, middle ear, and sinuses; larynx; lung and bronchus; pleura; trachea, mediastinum, and other respiratory); cancers of the mesothelioma; bones and joints; and soft tissue, including heart; skin cancers, including melanomas and other non-epithelial skin cancers; Kaposi's sarcoma and breast cancer; cancer of the female genital system (cervix uteri; corpus uteri; uterus, ovary; vagina; vulva; and other female genital); cancers of the male genital system (prostate gland; testis; penis; and other male genital); cancers of the urinary system (urinary bladder; kidney and renal pelvis; ureter; and other urinary); cancers of the eye and orbit; cancers of the brain and nervous system (brain; and other nervous system); cancers of the endocrine system (thyroid gland and other endocrine, including thymus); lymphomas (Hodgkin's disease and non-Hodgkin's lymphoma), multiple myeloma, and/or leukemias (lymphocytic leukemia; myeloid leukemia; monocytic leukemia; and other leukemias).

## Claims

1. A method of manufacturing pharmaceutical composition for treating cancer, comprising
obtaining mononuclear cells from a subject with cancer;
contacting the mononuclear cells with a first culturing medium comprising IL-15, IL-12 and IL-18 for about 1-6 day(s) to obtain a cultured cell population; and
contacting the cultured cell population with a second culturing medium comprising IL-15 and IL-12 for about 1-6 day(s) after contacting with the first culturing medium;
wherein a negative selection of CD3⁻CD19⁻CD14⁻ is not performed on the mononuclear cells prior to the contact step.

2. The method of claim 1, wherein the mononuclear cells are derived from peripheral blood, cord blood or bone marrow.

3. The method of claim 1 or 2, wherein the first culturing medium and the second culturing medium further comprises a hematopoietic cell medium.

4. The method of claim 3, wherein the hematopoietic cell medium comprises AIM-V medium.

5. The method of claim 3 or 4, wherein the first culturing medium further comprises a serum protein.

6. The method of claim 5, wherein the serum protein comprises a human platelet lysate.

7. A pharmaceutical composition for treating cancer, comprising
(a) a modified nature killer (NK) cell with a phenotype of CD3⁻CD19⁻CD14⁻ CD56^{hi}CD16^{dim}NKG2D⁺CD11c⁺CD86⁺HLA-DR⁺CD83⁻;
(b) an antigen-specific T cell with a phenotype of 4-1BB⁺CD8⁺; and
(c) a pharmaceutically acceptable carrier or excipient.

8. The pharmaceutical composition of claim 7, wherein the NK cell and the antigen-specific T cell are generated by any one of the method of claims 1-6.

9. A pharmaceutical composition according to any of claims 7 and 8, for use in a method of treating cancer, comprising
administering an effective amount of the pharmaceutical composition of claim 7 or 8 to a subject in need thereof.

10. The pharmaceutical composition for use of claim 9, wherein the effective amount is about l×10³ to about l×10⁹ cells per dose.

11. The pharmaceutical composition for use of claim 9 or 10, wherein the modified NK cell or the antigen-specific T cell is autologous or allogeneic.

12. The pharmaceutical composition for use of claim 9, 10 or 11, wherein the modified NK cell or the antigen-specific T cell is derived from peripheral blood, cord blood or bone marrow.

13. The pharmaceutical composition for use of claim 9, 10, 11 or 12, wherein the cancer comprises cancer of the oral cavity and pharynx (lip, tongue, salivary gland, floor of mouth, gum and other mouth, nasopharynx, tonsil, oropharynx, hypopharynx, other oral/pharynx); cancers of the digestive system (esophagus; stomach; small intestine; colon and rectum; anus, anal canal, and anorectum; liver; intrahepatic bile duct; gallbladder; other biliary; pancreas; retroperitoneum; peritoneum, omentum, and mesentery; other digestive); cancers of the respiratory system (nasal cavity, middle ear, and sinuses; larynx; lung and bronchus; pleura; trachea, mediastinum, and other respiratory); cancers of the mesothelioma; bones and joints; and soft tissue, including heart; skin cancers, including melanomas and other non-epithelial skin cancers; Kaposi's sarcoma and breast cancer; cancer of the female genital system (cervix uteri; corpus uteri; uterus, ovary; vagina; vulva; and other female genital); cancers of the male genital system (prostate gland; testis; penis; and other male genital); cancers of the urinary system (urinary bladder; kidney and renal pelvis; ureter; and other urinary); cancers of the eye and orbit; cancers of the brain and nervous system (brain; and other nervous system); cancers of the endocrine system (thyroid gland and other endocrine, including thymus); lymphomas (Hodgkin's disease and non-Hodgkin's lymphoma), multiple myeloma, and/or leukemias (lymphocytic leukemia; myeloid leukemia; monocytic leukemia; and other leukemias).
